# EUROPEAN PATENT APPLICATION

(11) **EP 4 451 208 A1**
(43) Date of publication of application: **23.10.2024**
(21) Application number: 23169178.3
(22) Date of filing: 21.04.2023
(51) Int. Cl.: G06T 7/00, G06T 7/32, G06T 7/33, G06T 7/37, A61B 6/00, A61B 5/00

(54) **METHOD AND SYSTEM FOR PROVIDING A MERGED IMAGE OF AN ORAL CAVITY**

(71) Applicant: Siemens Healthineers AG, 91301 Forchheim (DE); Sirona Dental Systems GmbH, 64625 Bensheim (DE); Dentsply Sirona Inc., York, PA 17401 (US)
(72) Inventor: Braun, Tim, 64521 Groß-Gerau (DE); Burzan, Kim, 69488 Birkenau (DE); Greiser, Andreas, 91054 Erlangen (DE); Lauer, Lars, 91077 Neunkirchen (DE); Ulrici, Johannes, 64285 Darmstadt (DE); Wundrak, Stefan, 64625 Bensheim (DE)
(74) Representative: Siemens Healthineers Patent Attorneys

(57) **Abstract**

The invention relates to a computer-implemented method for providing merged image data of an object (32) inside an oral cavity of a patient (15). The invention also relates to a magnetic resonance system (10), comprising a magnetic resonance device, an optical imaging device (40), a control unit (22) and a processing unit (28), wherein the control unit (22) is configured to control the magnetic resonance device to acquire magnetic resonance image data from an object (32) inside an oral cavity of a patient (15) and/or to control the optical imaging device to acquire optical data from the object (32) inside the oral cavity of the patient (15), wherein the processing unit (28) is configured to merge the optical data with the magnetic resonance image data.

## Description

The invention relates to a computer-implemented method and a system for providing merged image data of an object inside an oral cavity of a patient.

When applying magnetic resonance imaging in dentistry, hard structures of teeth (e. g. dentin, enamel) typically provide a poor signal for echo-time regimes that are most commonly available in clinical magnetic resonance devices. As a result, the geometry of the teeth cannot be reasonably well derived from magnetic resonance images. This constitutes a deficiency of magnetic resonance imaging in comparison to other imaging modalities, such as DVT (digital volume tomography) or X-Ray.

Specialized magnetic resonance scanners and sequences capable of imaging in ultra-short echo-time regimes have been used in scientific studies and the feasibility of imaging both enamel and dentin has been demonstrated. In alternative investigations, a dental impression material (e. g. a bite splint, toothpaste, or the like) inserted into the oral cavity of a patient has been used to indirectly depict surfaces of the teeth by visualizing the dental impression material.

However, due to various challenges such as high imaging effort, but also patient discomfort, none of the above-mentioned options is likely to be adopted for broader clinical practice in the near future.

It is therefore an objective of the invention to improve an efficiency of dental imaging applications based on magnetic resonance imaging.

This objective is achieved by a computer-implemented method and a medical imaging system according to the invention. Further advantageous embodiments are specified in the dependent claims.

Independent of the grammatical term usage, individuals with male, female or other gender identities are included within the term.

According to the inventive computer-implemented method, merged image data of an object inside an oral cavity of a patient is provided based on optical data of the oral cavity and magnetic resonance image data of a volume comprising the object.

The inventive computer-implemented method for providing merged image data of an object inside an oral cavity of a patient comprises a plurality of steps.

In one step of the inventive method, optical data of the oral cavity comprising surface information of the object is acquired.

Preferably, the optical data is acquired from an optical imaging device, such as a camera, a digital camera, an infrared camera, an optical profiler, a 3D scanner, or a combination of such optical imaging devices.

It is also conceivable that the optical data is acquired from a storage unit, such as a local data storage (i. e. a hard disk drive, a solid-state drive, a flash drive, etc.), a network storage device, a cloud storage, or the like.

In a preferred embodiment, the acquired optical data of the oral cavity has a spatial resolution of at least 60 pm, at least 40 µm or at least 20 pm. In one embodiment, the spatial resolution of the optical data may correspond to a slice thickness of the magnetic resonance image data. In using optical data with a spatial resolution in the specified range, the spatial resolution may be on par or at least in a comparable range to the spatial resolution of the magnetic resonance image data. Thus, the merging of the optical data with the magnetic resonance image data may be facilitated and/or a quality of the merged image data may be improved.

Optionally, the inventive method may comprise the step of capturing the optical data of the oral cavity via an optical imaging device. However, the optical data may also be retrieved from a storage unit as described above.

The object comprises soft-tissue, such as blood vessels, nerves, a pulp, or the like. The object may also comprise hard-tissue, such as enamel and/or dentin. For example, the object may represent a tooth, a plurality of teeth, a dental arch or both dental arches of the patient.

According to an embodiment, the acquiring of optical data of the oral cavity comprises acquiring optical image data of an upper and/or a lower jaw of the patient.

In a further step of the inventive method, magnetic resonance image data of a volume of the oral cavity comprising the object is acquired. The acquired magnetic resonance image data contains soft-tissue information of the object.

In a preferred embodiment, the inventive method comprises the step of capturing or recording the magnetic resonance image data via a magnetic resonance imaging device. However, the magnetic resonance image data or a part of the magnetic resonance image data may also be retrieved from a storage unit as described above.

According to a further step of the inventive method, the optical data is merged with the magnetic resonance image data of the object to provide merged image data of the object.

The merging of the optical data with the magnetic resonance image data may comprise adapting, modifying, combining and/or superimposing image information contained within the optical data and the magnetic resonance image data. The image information may relate to color values, grey values and/or contrasts of individual pixels, but also groupings or arrays of pixels, which may indicate tissue structures, anatomies, or the like. However, an image information may also relate to an image parameter, such as an image resolution and/or an image scale. The optical data and the magnetic resonance image data may comprise any data type suitable for (digitally) storing and/or processing the image data. In one embodiment, the optical data and/or the magnetic resonance image data comprise raw or unprocessed image data. However, the optical data and/or the magnetic resonance image data may also comprise one or more (processed) digital images, such as an optical image and/or a magnetic resonance image.

In one embodiment, the merging of the optical data and the magnetic resonance image data comprises scaling the magnetic resonance image to the optical image. It is also conceivable that the merging of the optical data with the magnetic resonance data comprises assigning pixels of an optical image to a magnetic resonance image or vice versa.

In a preferred embodiment, the merging of the optical data and the magnetic resonance image data comprises transforming the optical data and the magnetic resonance image data into one coordinate system. This process may also be understood as registering the optical data (or an optical image based on the optical data) and the magnetic resonance image data (or a magnetic resonance image based on the magnetic resonance image data).

Preferably, a processing unit configured to process image data is used to merge the optical data and the magnetic resonance image data. The processing unit may comprise a dedicated interface configured for receiving the optical data and/or the magnetic resonance image data.

The merged image data may comprise portions of the optical data and/or portions of the magnetic resonance image data.

In a preferred embodiment, the optical data and the magnetic resonance image data comprise volumetric data, i. e. a 3D representation of the object within the oral cavity of the patient. However, the optical data may also comprise 2D data. In this case, merging the optical data and the magnetic resonance data may comprise mapping a 2D representation of the object onto a 3D magnetic resonance image or a 3D model of the object based on the magnetic resonance image data.

In a further step of the inventive method the merged image data is output. Outputting the merged image data may comprise storing the merged image in a storage unit according to an embodiment described above. However, outputting the merged image data may also comprise displaying a merged image based on the merged image data via a dedicated display unit, such as a screen or monitor, a display unit of a mobile device (i. e. tablet, smartphone, etc.), or the like. It is also conceivable that the merged image data is output to a further processing application, such as an image viewer or an image-processing application.

The inventive method favorably allows for a provision of diagnostic images of a part of the dentition of a patient based on magnetic resonance imaging while avoiding costly modifications to the magnetic resonance imaging device, such as dedicated radio-frequency coils for dental imaging or changes to a radio-frequency system.

Furthermore, the inventive method may favorably allow for reduced scan times in comparison to conventional magnetic resonance devices using imaging sequences adapted for imaging of hard-tissue (in addition to a disease specific imaging sequence), such as ultra-short echo-time (UTE) sequences. For example, the dedicated magnetic resonance imaging sequence may favorably be replaced by a fast optical scan of the oral cavity providing the optical data.

The inventive method may also allow for increased patient comfort in comparison to dental imaging applications which require the placement of a dental impression material within the oral cavity of the patient during the magnetic resonance measurement.

According to an embodiment of the inventive method, the merging of the optical data with the magnetic resonance image data comprises registering the optical data with the magnetic resonance image data.

In one example, the optical image is merged with the magnetic resonance image. The merging of the optical image with the magnetic resonance image may be carried out by means of any image registration method, such as an area-based method and/or a feature-based method.

It is conceivable, that the image registration method is based on a correlation function, a correspondence of control points, a global and/or local transformation, a pattern recognition, a radial basis function, a Fourier transform, or the like. The image registration method can be complemented using optical and/or magnetic markers, orientation points and/or a geometric equivalence of the positioning of the examination object relative to the magnetic resonance apparatus. Furthermore, registering the optical data with the magnetic resonance image data may comprise using a body model. For example, the body model may be configured to account for a relative movement between the lower jaw and the upper jaw during the acquisition of the magnetic resonance image data with the magnetic resonance imaging device. The registering of the optical data with the magnetic resonance image data may also be based on or carried out via an AI algorithm.

By registering the optical data with the magnetic resonance data, an increased spatial accuracy of the magnetic resonance image and/or an improved representation of a shape or outline of the object based on the magnetic resonance image data may favorably be provided.

According to a further embodiment of the inventive method, the optical data and the magnetic resonance image data comprise surface information of a section of a gingiva. The merging of the optical data with the magnetic resonance image data comprises matching the surface of the section of the gingiva in the magnetic resonance image data with the surface of the section of the gingiva in the optical data.

Due to its high color contrast with respect to enamel, but also its soft-tissue content, the gingiva may provide excellent contrasts and/or discernability in the magnetic resonance image as well as the optical image. Thus, the gingiva may favorably be used in a feature-based image registration or an image registration method comprising pattern recognition.

In a preferred embodiment, the optical data and the magnetic resonance image data comprise surface information of a section of a gum line. The surface information may comprise color values and/or grey values assigned to coordinates in a 3D space. Due to its excellent discernability from enamel, a shape or contour of the gum line may be determined in both the optical image as well as the magnetic resonance image.

The merging of the optical data with the magnetic resonance image data comprises matching the section of the gum line in the magnetic resonance image data with the section of the gum line in the optical data. Matching the section of the gum line in the magnetic resonance image data with the section of the gum line in the optical data may comprise scaling the magnetic resonance image based on the optical image. However, it is also conceivable that the optical image is scaled in dependence of the magnetic resonance image.

In one embodiment, a cost function or minimizing function may be used for scaling of the magnetic resonance image based in the optical image (or vice versa). For example, the magnetic resonance image may be scaled in such a way that a deviation between one or more reference dimensions of the gum line in the magnetic resonance image and the gum line in the optical image is minimized or falls below a predetermined threshold.

In using a section of the gingiva and/or a section of the gum line for the merging of the optical data with the magnetic resonance image data, an accuracy of matching the optical image with the magnetic resonance image may favorably be increased.

According to a further embodiment of the inventive method, the object is a tooth. The acquiring of magnetic resonance image data of the oral cavity comprises acquiring magnetic resonance image data of a pulp of the tooth. As the pulp of the tooth comprises soft-tissue, it may provide for an excellent magnetic resonance contrast and discernability from a surrounding dentin and/or enamel.

The merging of the optical data with the magnetic resonance image data comprises determining a shape of the tooth based on the magnetic resonance image data of the pulp in dependence of a biogeneric model and matching the surface of the tooth from the biogeneric model with a shape of the tooth in the optical data.

The pulp is contained within a pulp chamber typically mimicking an overall shape of the tooth. The natural similarity between the shape of the pulp chamber and the shape of the tooth may be used for determining the shape of the tooth based on the shape of the pulp chamber in the magnetic resonance image via the biogeneric model.

The shape of the tooth determined via the biogeneric model may be used for the merging of the optical data with the magnetic resonance image data, particularly for matching a feature in the optical image with the shape of the tooth provided via the biogeneric model.

In one embodiment, the magnetic resonance image may be positioned and/or scaled relative to the optical image based on a fit between the shape of the tooth in the optical image and the shape of the tooth determined via the biogeneric model. For example, a cost function or minimizing function may be used for positioning and/or scaling of the magnetic resonance image relative to the optical image.

By using a biogeneric model for determining the shape of a tooth or a plurality of teeth, additional imaging sequences for visualizing dentin and/or enamel may favorably be avoided. Furthermore, an accuracy of the merged image data may favorably be improved.

In one embodiment of the inventive method, the merging of the optical data with the magnetic resonance image data comprises a distortion correction of the magnetic resonance image data based on the optical data and/or a scaling of the magnetic resonance image data in dependence of the optical data.

The object depicted in the magnetic resonance image may be warped or deformed in comparison to the object in the optical image. Thus, the magnetic resonance image may be positioned, scaled and/or deformed in such a way that the shape of the object in the magnetic resonance image matches the shape of the object in the optical image. It is conceivable that distortion and/or scaling effects present in the magnetic resonance image data may be corrected or at least adjusted with respect to any distortion and/or scaling effects present in the optical image.

For example, by matching the optical image with the magnetic resonance image based on the surface information of a section of a gingiva, a section of the gum line and/or the shape of the tooth, an accurate fit between the optical image and the magnetic resonance image may be provided.

By adjusting or correcting the magnetic resonance image data in dependence of the optical data, diagnostically relevant regions contained in the magnetic resonance data may favorably be superimposed in and/or allocated to correct positions of the optical image when merging the optical data with the magnetic resonance image data. Thus, a risk of misplacing a diagnostically relevant region with respect to the optical image may favorably be reduced. Furthermore, an interpretation of the diagnostically relevant regions based on the merged image data may favorably be facilitated and/or a risk of misdiagnosis may favorably be reduced.

As a further advantage, distortion correction of the magnetic resonance image data according to the inventive method may allow for a provision of magnetic resonance images with increased spatial accuracy in comparison to conventional magnetic resonance images. Particularly, an increased geometrical accuracy across a magnetic resonance imaging volume may be obtained.

According to an embodiment, the inventive method comprises the further step of determining an information about a filling and/or a metal object inside the oral cavity based on the optical data and adapting an imaging protocol for acquiring of magnetic resonance image data of the oral cavity based on the information about the filling and/or the metal object.

For example, the information about the filling and/or metal object inside the oral cavity may comprise a shape, a dimension, a mass, a type, a location and/or a volume of the filling and/or metal object. The information about the filling and/or metal object may be acquired from the optical data, i. e. by processing the optical data via a pattern recognition algorithm and/or a dedicated image processing algorithm.

Adapting an imaging protocol for acquiring of the magnetic resonance image data may comprise adapting one or more imaging parameters. An imaging parameter may be any parameter effecting the acquisition of magnetic resonance image data. Examples of imaging parameters are a field of view, an echo-time, a repetition time, a shape and/or timing of a radiofrequency pulse and/or a sequence of radiofrequency pulses, but also a slice thickness, a slice increment, a gradient performance, and the like.

According to an embodiment, a deformation or deflection of a magnetic field in presence of a metal object is considered when adapting the imaging protocol for the acquiring of the magnetic resonance image data. Preferably, a later correction of the magnetic resonance image data based on the optical data is taken into account when adapting the imaging protocol. For example, a compatibility with the optical data (i. e. regarding resolution, field of view, slice orientation, or the like) may be considered when adapting the imaging protocol.

In adapting the imaging protocol used for acquiring the magnetic resonance image data, any requirements for correcting the magnetic resonance data based on the optical data and/or the merging of the optical data with the magnetic resonance image data may already be considered when acquiring the magnetic resonance image data. Particularly, detrimental effects of metal objects inside the oral cavity on a quality of the magnetic resonance image data and/or the merged image data may favorably be reduced. Thus, a high quality and/or high accuracy of the merged image data may be provided.

According to a further embodiment of the inventive method, the acquiring of optical data of the oral cavity comprises acquiring optical image data of an upper and/or a lower dental arch of the patient.

In a preferred embodiment, the acquiring of optical data of the oral cavity comprises acquiring optical image data of both the upper dental arch and lower dental arch of the patient. Likewise, the magnetic resonance image data may be acquired from a volume comprising the upper dental arch and/or the lower dental arch of the patient. In merging the optical data with the magnetic resonance image data, merged data of the upper dental arch and lower dental arch, i. e. an entire dentition of the patient, may favorably be provided.

According to an embodiment, the acquiring of optical data and the acquiring of magnetic resonance image data is restricted to the upper dental arch, the lower dental arch, a section of the upper dental arch, or a section of the lower dental arch. In limiting the acquiring of optical data and/or the acquiring of magnetic resonance image data to a dental arch or a section of a dental arch (i. e. a set of teeth or a tooth), a time required for acquiring of the optical data and/or the magnetic resonance image data may favorably be reduced while still providing merged image data of a diagnostically relevant region.

According to a further embodiment of the inventive method, the merging of the optical data with the magnetic resonance image data comprises generating a contour information of a tooth and/or a periodontium based on the optical data and merging said contour information with soft-tissue information in the magnetic resonance image data.

The contour information may be understood as 3D surface information. For example, the contour information may comprise a 3D representation of a structure, such as a tooth, a set of teeth, a dental arch or both dental arches of a patient. Particularly, the contour information may comprise a 3D surface curve or contour plot comprising information on the shape of the tooth and/or periodontium.

Generating the contour information may comprise establishing a 3D contour plot or surface curve of the tooth, the set of teeth, the one or more dental arches and/or a section of the periodontium in a coordinate system. Thus, the contour information can be merged with the acquired magnetic resonance image data comprising soft-tissue information of said structures. For example, contour information can be converted into coordinates of the magnetic resonance image data by a coordinate transformation, i. e. using a transformation function or a transformation matrix.

Furthermore, generating the contour information may comprise using image processing algorithms, such as photogrammetry, but also additional sensors (i. e. infrared sensors, LIDAR sensors, or the like) providing depth information, when acquiring the optical data.

In one embodiment, one or more landmarks and/or anatomical structures in the magnetic resonance image data and the optical data are used to calculate a transformation matrix, which converts the coordinate system of the magnetic resonance image data and the coordinate system of the optical data into one another.

According to a further embodiment of the inventive method, the outputting of the merged image data comprises storing the merged image data in a storage unit and/or displaying the merged image data.

For example, the merged image data may be output to a local storage unit of the magnetic resonance imaging device, a network storage connected to the magnetic resonance device and/or a cloud storage. Thus, the merged image data may favorably be accessed by different terminal devices such as a display, a monitor, a medical device (i. e. a further magnetic resonance imaging device or a different medical device), a treatment planning system, but also a mobile device, such as a tablet, a smart phone, and the like.

In a preferred embodiment, the outputting of the merged image data comprises integrally outputting the contour information and the soft-tissue information.

The merged image data may comprise soft tissue information from the magnetic resonance image data as well as contour information from the optical data, thus favorably compensating for a lack of hard-tissue information in the magnetic resonance image data, while allowing for reduced magnetic resonance measurement times in comparison to conventional magnetic resonance devices providing a contrast for hard tissues.

Furthermore, the merged image data may favorably provide for an integrated visualization of the contour information and the soft tissue information, thus facilitating diagnostic interpretation by trained personnel and/or reducing a risk of a misdiagnosis.

In a further embodiment of the inventive method, the merging of the optical data with the magnetic resonance image data comprises assigning image information of the magnetic resonance image data to sections of the optical data.

Merging the optical data with the magnetic resonance image data may comprise generating a data structure. The data structure may comprise a part of the optical data, a part of the magnetic resonance data as well as image information. The image information may comprise any information related to the optical image and/or the magnetic resonance image, particularly settings and/or parameters used to acquire the optical data and/or magnetic resonance image data.

In a preferred embodiment, the image information comprises one or more slice positions of the magnetic resonance image data. Thus, the outputting of the merged image data may comprise outputting a slice position in dependence of a selection of a section of the optical data.

The image information may be assigned to one or more sections of the optical image, the magnetic resonance image and/or a merged image based on the merged image data. For example, one or more slice positions of the magnetic resonance measurement carried out to acquire the magnetic resonance image data are assigned to corresponding sections of the optical image. Thus, the merged image may favorably allow for navigating through a slice stack by selecting a desired section in the optical image and/or the merged image in a dedicated user interface.

In one embodiment, outputting the merged image data may comprise integrally outputting the merged image data and the image information. The merged image data and the image information may be included in a dedicated data structure. The merged image data and the image information may be output to an image viewer, a treatment planning system, a diagnosis system, or the like.

According to an embodiment, the merged image data and the image information is output to a user interface enabling the user to perform operations, such as sorting, filtering, selecting, and/or modifying the merged image, the optical image and/or the magnetic resonance image in dependence of the image information.

The inventive method may favorably enable a user to navigate the merged image data and/or modify the merged image in dependence of the image information.

The inventive magnetic resonance system comprises a magnetic resonance device, an optical imaging device, a control unit and a processing unit. The control unit is configured to control the magnetic resonance device to acquire magnetic resonance image data from an object inside an oral cavity of a patient and/or to control the optical imaging device to acquire optical data from the object inside the oral cavity of the patient. The processing unit is configured to merge the optical data with the magnetic resonance image data and to provide merged image data of the object.

The magnetic resonance system, particularly the control unit and/or processing unit, may comprise one or more data interfaces configured for receiving or acquiring the optical data and the magnetic resonance image data. For example, the processing unit may be connected to the optical imaging device via a suitable signal connection (i. e. an electrical signal connection or a wireless connection).

The control unit may represent a main control unit of the magnetic resonance device or an independent control unit. The control unit may be connected to the optical imaging device via a suitable signal connection. In case the control unit is an independent component, the control unit may be connected to the main control unit of the magnetic resonance device.

The processing unit may represent a main processing unit of the magnetic resonance device or an independent processing unit. The processing unit may be connected to the optical imaging device via a suitable signal connection. In case the processing unit is an independent component, the processing unit may be connected to the main control unit of the magnetic resonance device via a suitable signal connection. It is conceivable that the processing unit is incorporated or integrated within the control unit.

The processing unit and the control unit are configured for coordinating and/or carrying out an inventive method according to an embodiment described above.

The processing unit and/or the control may comprise any graphics unit, storage unit and/or further component required for carrying out the inventive method according to an embodiment described above.

In one embodiment, the processing unit is configured to process the optical data, the magnetic resonance image data and/or the merged image data. The processing unit may implement an image processing algorithm, a pattern recognition algorithm, or the like for processing the optical data, the magnetic resonance image data, and/or the merged image data according to an embodiment described above.

The inventive magnetic resonance system shares the advantages of the inventive method. Furthermore, the inventive magnetic resonance system may favorably allow for carrying out an embodiment of the inventive method in a robust and/or reproduceable manner.

According to an embodiment, the magnetic resonance system according comprises an output unit configured for outputting merged image data to a user, and a user interface configured for adapting a magnetic resonance image and/or a merged image based on the magnetic resonance image data in dependence of an interaction with an optical image based on the optical data.

Preferably, the user interface is configured to allow for sorting, filtering, selecting, navigating and/or modifying the magnetic resonance image and/or the merged image in dependence of a user-selection of a section in the optical image. For example, the user interface may be configured to provide and/or output a section of the magnetic resonance image and/or an image information related to a section of the magnetic resonance image when a section in the optical image is selected via the user interface.

The inventive magnetic resonance system may favorably allow for synchronizing of operations performed on the optical image and the merged image and/or the magnetic resonance image. Furthermore, operations and/or selections regarding the optical image may favorably be applied to the magnetic resonance image and/or the merged image.

Further advantages and details of the present invention may be recognized from the embodiments described below as well as the drawings. The figures show:
- Fig. 1: an embodiment of an inventive magnetic resonance system,
- Fig. 2: a schematic representation of an embodiment of an inventive magnetic resonance system,
- Fig. 3: a schematic representation of an optical image of an oral cavity,
- Fig. 4: a schematic representation of a merged image of an oral cavity,
- Fig. 5: a flow chart of an embodiment of an inventive method for providing merged image data of an object inside an oral cavity of a patient.

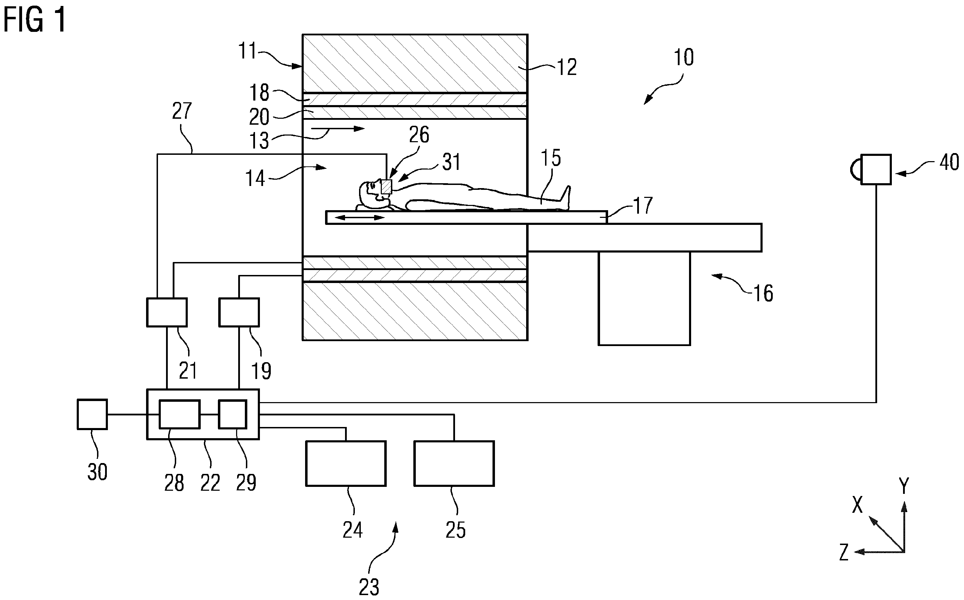

Fig. 1 shows an embodiment of a magnetic resonance system 10 according to the invention. In the depicted example, the magnetic resonance system 10 comprises a magnetic resonance device configured for acquiring magnetic resonance image data from an object positioned within an imaging region 14. In the depicted example, the object is a patient 15. The magnetic resonance device comprises a field generation unit 11 including a cylindrical magnet 12 that provides a homogenous, static magnetic field 13 (B0 field). The imaging region 14 may be defined by a bore configured for accommodating the object during an imaging examination or magnetic resonance measurement. The imaging region 14 is surrounded or enclosed by the field generation unit 11 in a circumferential direction.

Preferably, the magnetic resonance system 10 comprises a patient support 16 or patient table configured for transporting the patient 15 into the imaging region 14, particularly an isocenter (not shown) within the imaging region 14.

The magnetic resonance system 10 may comprise a gradient system 18 configured for providing magnetic gradient fields used for spatial encoding of magnetic resonance signals acquired during a magnetic resonance measurement. The gradient system 18 is activated or controlled by a gradient controller 19 via an appropriate current signal. It is conceivable that the gradient system 18 comprises one or more gradient coils for generating magnetic gradient fields in different, preferably orthogonally oriented, spatial directions.

The magnetic resonance system 10 may further comprise a high frequency system including a radiofrequency antenna 20 (body coil), which may be integrated into the magnetic resonance device. The radiofrequency antenna 20 is operated via a radiofrequency controller 21 that controls the radiofrequency antenna 20 to generate a high frequency magnetic field and emit radiofrequency excitation pulses into the imaging region 14.

The magnetic resonance system 10 may further comprise a local coil 26, which is positioned in proximity to a diagnostically relevant region (i. e. a head region or a jaw region) of the patient 15. The local coil 26 may be configured to emit radiofrequency excitation pulses into the patient 15 and/or receive magnetic resonance signals from the patient 15. It is conceivable, that the local coil 26 is controlled via the radiofrequency controller 21.

In the depicted embodiment, the local coil 26 is a dental coil, which is positioned in proximity to a jaw region 31 of the patient 15. Preferably, the dental coil comprises one or more antenna elements (not shown) configured to receive magnetic resonance signals from the jaw region 31 of patient 15 and transmit the detected magnetic resonance signals to the processing unit 28 and/or the control unit 22. In the illustrated example, the dental coil is connected to the radiofrequency unit 21 via a suitable signal connection 27. The dental coil 26 may be configured to emit radiofrequency signals into the jaw region 31 of patient 15 to excite nuclear spins.

According to a preferred embodiment, the dental coil is a mask configured to be positioned on a skin surface of the jaw region 31 of patient 15. However, it is also conceivable that the dental coil 26 is mechanically connected to a mouthpiece which is positioned within the oral cavity of the patient 15.

The magnetic resonance system 10 preferably comprises an optical imaging device 40, such as a 2D camera, a 3D camera, an infrared camera, or the like. The optical imaging device 40 is designed to acquire optical data of the oral cavity of the patient 15. For acquiring of the optical data, the patient 15 may be positioned in front of the optical imaging device 40 before being positioned in the imaging region 14. However, it is also conceivable that the optical imaging device 40 is positioned within the imaging region 14 or adjacent to the imaging region 14. In one embodiment, the optical imaging device 40 is connected to the local coil 26.

The magnetic resonance system 10 may further comprise a control unit 22 configured for controlling the magnetic resonance device. The control unit 22 may include a processing unit 28 configured for processing magnetic resonance image data and reconstructing magnetic resonance images. The processing unit 28 may also be configured to process input from a user of the magnetic resonance system 10 and/or provide an output to the user. For this purpose, the processing unit 28 and/or the control unit 22 can be connected to an output unit 24 and an input unit 25 via a suitable signal connection.

For a preparation of a magnetic resonance imaging measurement, preparatory information, such as imaging parameters or patient information, can be provided to the user via the output unit 24. Preferably, the output unit 24 is configured to output control information, such as imaging parameters of the magnetic resonance measurement. The output unit 24 may also be configured to output a magnetic resonance image, an optical image and/or a merged image of the oral cavity of the patient 15. The input unit 25 may be configured to receive information and/or imaging parameters from the user. However, the input unit 25 may also be configured to enable the user to select a section of the optical image, but also to navigate and/or modify the optical image, the merged image and/or the magnetic resonance image.

The magnetic resonance system 10 may comprise a user interface 23 comprising the output unit 24 and the input unit 25. The user interface 23 be configured to enable the user to control an acquisition of magnetic resonance image data via the magnetic resonance device and/or an acquisition of optical data via the optical imaging device 40. Furthermore, the user interface may enable the user to interact with the magnetic resonance image, the optical image and/or the merged image. Preferably, the user interface 23 is configured to allow for an interaction with a magnetic resonance image and/or a merged image in dependence of an interaction with an optical image. For example, the user interface may be configured to enable the user to navigate through a stack of slices of the magnetic resonance image data in dependence of a selection of a structure in the optical image. However, the user interface may be configured for allowing a user to interact with a merged image and/or a magnetic resonance image based on the magnetic resonance image data in dependence of an interaction with an optical image based on the optical data.

As illustrated in Fig. 1, the processing unit 28 may be connected to a storage unit 29 of the magnetic resonance device. Optionally, the processing unit 28 may be connected to a cloud 30. The processing unit 28 may be configured to store data, such as optical data, magnetic resonance image data, and/or merged image data, on the storage unit 29 and/or the cloud 30 (i. e. a cloud storage). However, the processing unit 28 may also be configured to acquire such data from the storage unit 29 and/or the cloud 30 by means of a suitable interface (not shown).

It is conceivable that some processing tasks are outsourced to the cloud 30 (i. e. a cloud processor). For example, the cloud 30 may be configured to receive acquired optical data and magnetic resonance image data and merge the optical data with the magnetic resonance image data to provide merged image data of the object according to an embodiment described herein.

In a further example, the cloud 30 is configured to determine a shape of a tooth based on the magnetic resonance image data of a pulp in dependence of a biogeneric model. The cloud 30 may also be configured to match a surface of the tooth from the biogeneric model with a shape of the tooth in the optical data.

Preferably, the cloud 30 is configured to transmit a result of a processing task to the processing unit 28.

The magnetic resonance system 10 may of course comprise further components usually associated with magnetic resonance devices. It is conceivable that the field generation unit 11 of the magnetic resonance device has a conventional cylindrical design or a C-shaped design. However, the field generation unit 11 may also exhibit a triangular design, or an asymmetrical design. Particularly, the magnetic resonance system 10 may be configured to perform a magnetic resonance measurement of a standing or sitting patient 15.

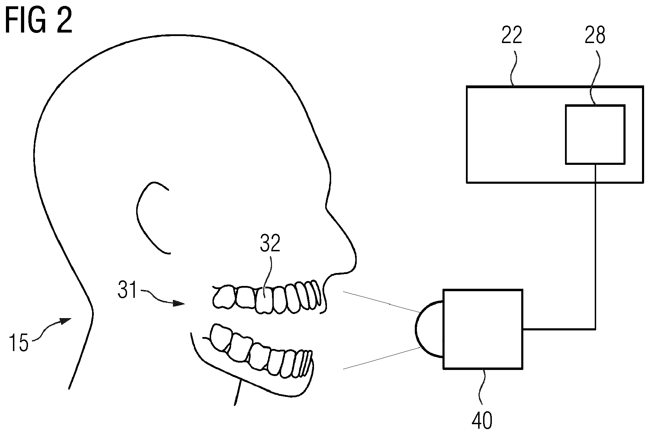

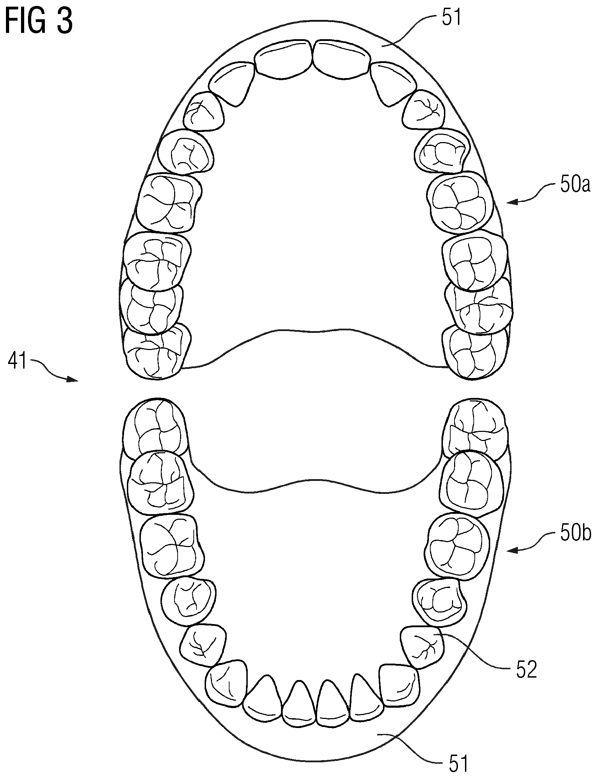

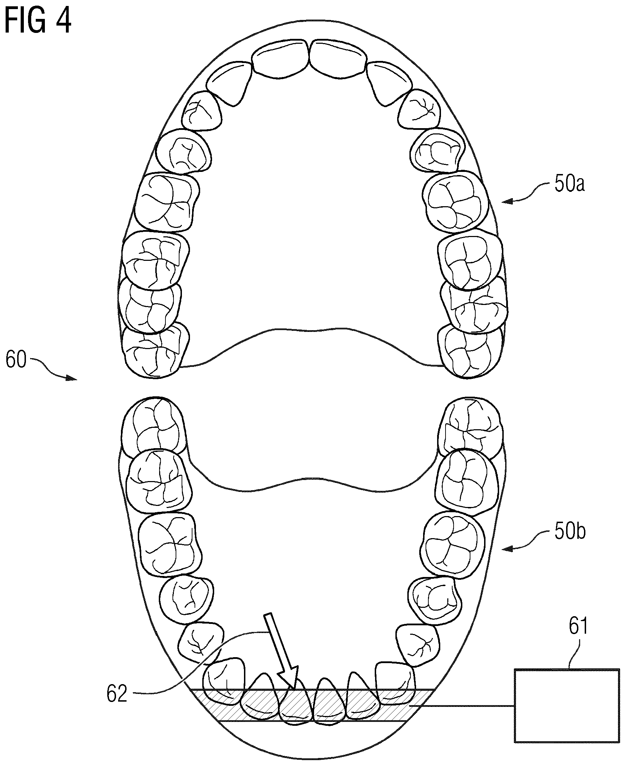

Fig. 2 shows an embodiment of the magnetic resonance system 10 wherein a camera 40 is positioned in front of the oral cavity of the patient 15 for receiving optical data of an object inside the oral cavity. The mouth of the patient 15 is open so that the camera 40 can capture optical data of an object 32 within the oral cavity, such as a tooth, a set of teeth, an upper and/or lower jaw, or the entire dentition of the patient 15 (see Figs. 3 and 4). The camera is connected to the processing unit 28 and/or the control unit 22 via a suitable signal connection. Thus, the camera 40 may transmit the acquired optical data of the object 32 to the processing unit 28. However, the optical imaging device 40 may also be configured to transmit the acquired optical data to the storage unit 29 and/or the cloud 30.

Fig. 3 shows an example of an optical image 41 acquired via the optical imaging device 40. In the depicted example, both the lower dental arch 41b and the upper dental arch 50a are shown. However, the optical image 41 may be restricted to just the lower dental arch 50b, the upper dental arch 50a, or even a tooth or a set of teeth belonging to a dental arch 50.

The optical image 41 depicted in Fig. 3 comprises sections of the gingiva 51 which provide an excellent color contrast with respect to the enamel 52 of the teeth. This color contrast may be used, for example, in a feature-based image registration or an image registration method comprising pattern recognition according to an embodiment described above.

Fig. 4 shows an embodiment of a merged image 60. In the illustrated example, the optical image is not superimposed with the magnetic resonance image. However, image information regarding the magnetic resonance image has been assigned to sections of the optical image 41 according to an embodiment described above. For example, a user may select a section in the optical image 41 (or merged image with just the optical image being visible) to trigger an interaction menu 61. The interaction menu 61 may display and/or enable the user to access or select a slice in the magnetic resonance image data that corresponds to the selected section in the optical image. The interaction menu 61 may also provide or display a section of the magnetic resonance image corresponding to the selected section in the optical image 41. As depicted in Fig. 4, a selected section of the optical image may be highlighted.

The interaction menu 61 may also be configured to indicate imaging information, such as a slice position and/or an imaging parameter of the magnetic resonance measurement. In a preferred embodiment, the interaction menu 61 is configured to allow the user to navigate through different slice positions, superimpose the magnetic resonance image, jump to a corresponding slice in the magnetic resonance image, change a transparency of the optical image and/or the magnetic resonance image, indicate an imaging parameter associated with the optical data and/or the magnetic resonance image data, and the like.

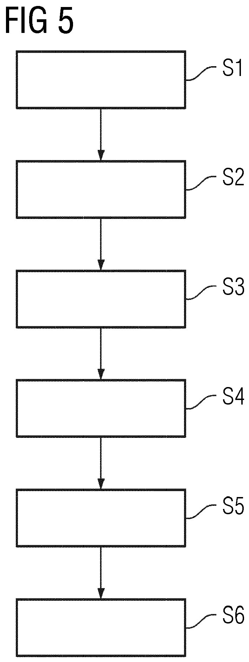

Fig. 5 shows a flow chart of an embodiment of the inventive method for providing merged image data 60 of an object inside an oral cavity of a patient 15.

In step S1, optical data of the oral cavity comprising surface information of the object is acquired. Preferably, the optical data is acquired via a high-resolution 3D camera 40 providing a spatial resolution of at least 60 pm, at least 40 pm, or at least 20 pm. The camera 40 may be connected to the magnetic resonance system 10 as illustrated in Figs. 1 or 2. However, the camera may also represent an independent component, i. e. located separately or remotely from the magnetic resonance system 10. In this case, acquiring the optical data of the oral cavity may comprise receiving the optical data via a cloud 30, a network storage, a local storage 29, or the like.

Preferably, the acquiring S1 of optical data of the oral cavity comprises acquiring optical image data of an upper dental arch 50a and/or a lower dental arch 50b of the patient 15 (see Fig. 3).

In a step S2, magnetic resonance image data of a volume of the oral cavity comprising the object is acquired. The magnetic resonance image data contains soft-tissue information of the object. The magnetic resonance image data may be acquired using a magnetic resonance system 10 as depicted in Fig. 1. However, it is also conceivable that the magnetic resonance image data is received via a cloud 30, a network storage, a local storage 29, or the like. For example, the inventive method may be carried out remotely from the magnetic resonance device used to acquire the magnetic resonance image data.

In one embodiment, the object is a tooth and the acquiring of magnetic resonance image data of the oral cavity comprises acquiring magnetic resonance image data of a pulp of the tooth.

In a further step S3, the optical data is merged with the magnetic resonance image data to provide merged image data of the object.

According to an embodiment, the merging of the optical data with the magnetic resonance image data comprises registering an optical image based on the optical data with a magnetic resonance image based on the magnetic resonance image data by means of an image registration method. The image registration method may be based on a correlation function, a correspondence of control points, a global and/or local transformation, a pattern recognition, a radial basis function, a Fourier transform, or the like.

According to a further embodiment, the optical data and the magnetic resonance image data comprise surface information of a section of a gingiva 51. In such a case, the merging of the optical data with the magnetic resonance image data may comprise matching the surface of the section of the gingiva 51 in the magnetic resonance image data with the surface of the section of the gingiva 51 in the optical data. Preferably, a feature-based image registration or an image registration method comprising pattern recognition is used to register the optical data with the magnetic resonance image data.

In one embodiment, the optical data and the magnetic resonance image data comprise surface information of a section of a gum line and the merging of the optical data with the magnetic resonance image data comprises scaling the magnetic resonance image based on a representation of the gum line in the optical image 41 and the magnetic resonance image.

According to a further embodiment, the step of merging the optical data with the magnetic resonance image data comprises determining a shape of the tooth based on the magnetic resonance image data of the pulp in dependence of a biogeneric model and matching the shape of the of the tooth from the biogeneric model with a shape of the tooth in the optical data. Determining the shape of the tooth in dependence of the biogeneric model as well as matching the shape of the of the tooth from the biogeneric model with a shape of the tooth in the optical data may be carried out via the processing unit 28 or a processing unit in the cloud 30.

Preferably, the step of merging the optical data with the magnetic resonance image data comprises correcting a distortion of the magnetic resonance image data based on the optical data and/or a scaling of the magnetic resonance image data in dependence of the optical data. For example, the representations of the gingiva 51 and/or the gum line in the optical image and the magnetic resonance image may be used for determining and/or correction distortions in the magnetic resonance image data.

The step merging the optical data with the magnetic resonance image data may further comprise generating a contour information of a tooth and/or a periodontium based on the optical data and merging said contour information with soft-tissue information in the magnetic resonance image data. The contour information of the tooth and/or periodontium may be generated via the processing unit 28 or a processing unit in the cloud 30. For example, the contour information may be generated via photogrammetry. However, it is also conceivable that depth information from an additional sensor, such as an infrared sensor or LIDAR sensor (not shown in Fig. 1), is used to generate the contour information of the tooth and/or periodontium. Such an additional sensor may for a part of the magnetic resonance system 10 as depicted in Fig. 1 or the camera 40 as depicted in Fig.2.

In each embodiment, the step of merging the optical data with the magnetic resonance image data may comprise assigning image information of the magnetic resonance image data to sections of the optical data.

According to an optional step S4, an information about a filling and/or a metal object inside the oral cavity is determined based on the optical data. The information about the filling and/or metal object may be acquired from the optical data, i. e. by processing the optical data via a pattern recognition algorithm and/or a dedicated image processing algorithm. The optical data may be processed via the processing unit 28 or a processing unit in the cloud 30, which may be configured to implement said pattern recognition algorithm and/or dedicated image processing algorithm. The information about the filling and/or metal object inside the oral cavity may comprise a shape, a dimension, a mass, a type, a location but also a volume of the filling and/or metal object.

In an optional step S5, an imaging protocol for acquiring of magnetic resonance image data of the oral cavity is adapted based on the information about the filling and/or the metal object. For example, adapting the imaging protocol may comprise adapting one or more imaging parameters to account for a deformation or deflection of a magnetic field in proximity to the metal object.

In a step S6, the merged image data is output. Outputting the merged image data may comprise storing the merged image in the storage unit 29 or the cloud storage 30. However, outputting the merged image data may also comprise displaying the merged image 60 based on the merged image data via the output unit 24. The merged image data may also be output to a further processing application, such as an image viewer or an image-processing application and/or a terminal device, such as a tablet or smartphone.

In a preferred embodiment, outputting the merged image data comprises integrally outputting contour information based on the optical data and soft-tissue information based on the magnetic resonance image data. For example, outputting the merged image data may comprise outputting a merged image comprising a representation of the contour of teeth and/or the periodontium as well as a representation of a part of the pulp and/or a nerve. Integrally outputting contour information and soft-tissue information may also comprise superimposing the representation of the contour of teeth and/or the periodontium as well as the representation of the part of the pulp and/or nerve.

In a further example, outputting the merged image data comprises outputting a data structure comprising a part of the optical data, a part of the magnetic resonance data as well as image information.

The embodiments described above are to be recognized as examples. It is to be understood that individual embodiments may be extended by or combined with features of other embodiments if not stated otherwise. Particularly, the sequence of the steps of the inventive method is to be understood as an example. Individual steps may be carried out in a different order and/or overlap partially or completely in time.

## Claims

1. A computer-implemented method for providing merged image data of an object (32) inside an oral cavity of a patient (15), comprising the steps:
• acquiring optical data (S1) of the oral cavity, the acquired optical data comprising surface information of the object (32),
• acquiring magnetic resonance image data (S2) of a volume of the oral cavity comprising the object (32), the acquired magnetic resonance image data containing soft-tissue information of the object (32),
• merging (S3) the optical data with the magnetic resonance image data to provide merged image data of the object (32),
• outputting (S6) the merged image data.

2. The method according to claim 1, wherein the merging (S3) of the optical data with the magnetic resonance image data comprises registering the optical data with the magnetic resonance image data.

3. The method according to claim 1 or 2, wherein the optical data and the magnetic resonance image data comprise surface information of a section of a gingiva (51) and wherein the merging (S3) of the optical data with the magnetic resonance image data comprises matching the surface of the section of the gingiva (51) in the magnetic resonance image data with the surface of the section of the gingiva (51) in the optical data.

4. The method according to one of the preceding claims, wherein the optical data and the magnetic resonance image data comprise surface information of a section of a gum line and wherein the merging (S3) of the optical data with the magnetic resonance image data comprises matching the section of the gum line in the magnetic resonance image data with the section of the gum line in the optical data.

5. The method according to one of the preceding claims, wherein the object is a tooth, wherein the acquiring (S2) of magnetic resonance image data of the oral cavity comprises acquiring magnetic resonance image data of a pulp of the tooth and wherein the merging (S3) of the optical data with the magnetic resonance image data comprises determining a shape of the tooth based on the magnetic resonance image data of the pulp in dependence of a biogeneric model and matching the shape of the of the tooth from the biogeneric model with a shape of the tooth in the optical data.

6. The method according to one of the preceding claims, wherein the merging (S3) of the optical data with the magnetic resonance image data comprises a distortion correction of the magnetic resonance image data based on the optical data and/or a scaling of the magnetic resonance image data in dependence of the optical data.

7. The method according to one of the preceding claims, comprising the steps:
• determining (S4) an information about a filling and/or a metal object inside the oral cavity based on the optical data, and
• adapting (S5) an imaging protocol for acquiring (S2) of magnetic resonance image data of the oral cavity based on the information about the filling and/or the metal object.

8. The method according to one of the preceding claims, wherein the optical data of the oral cavity has a spatial resolution of at least 60 pm, at least 40 pm or preferably at least 20 pm.

9. The method according to one of the preceding claims, wherein the acquiring (S1) of optical data of the oral cavity comprises acquiring optical image data of an upper and/or a lower dental arch (50) of the patient (15).

10. The method according to any of the preceding claims, wherein the merging (S3) of the optical data with the magnetic resonance image data comprises generating a contour information of a tooth and/or a periodontium based on the optical data and merging said contour information with soft-tissue information in the magnetic resonance image data.

11. The method according to any of the preceding claim, wherein the outputting (S6) of the merged image data comprising storing the merged image data in a storage unit and/or displaying the merged image data.

12. The method according to the claims 10 and 11, wherein the outputting (S6) of the merged image data comprises integrally outputting the contour information and the soft-tissue information.

13. The method according to a preceding claim, wherein the merging (S3) of the optical data with the magnetic resonance image data comprises assigning image information of the magnetic resonance image data to sections of the optical data.

14. Magnetic resonance system (10), comprising a magnetic resonance device, an optical imaging device (40), a control unit (22) and a processing unit (28), wherein the control unit (22) is configured to control the magnetic resonance device to acquire magnetic resonance image data from an object (32) inside an oral cavity of a patient (15) and/or to control the optical imaging device (40) to acquire optical data from the object (32) inside the oral cavity of the patient (15), wherein the processing unit (28) is configured to merge the optical data with the magnetic resonance image data to provide merged image data of the object (32).

15. The magnetic resonance system according to claim 14, comprising an output unit (24) configured for outputting merged image data to a user, and a user interface (23) configured for interacting with a merged image and/or a magnetic resonance image based on the magnetic resonance image data in dependence of an interaction with an optical image based on the optical data.
